# EUROPÄISCHE PATENTSCHRIFT

(11) **EP 1 214 117 B1**
(45) Veröffentlichungstag und Bekanntmachung des Hinweises auf die Patenterteilung: **22.12.2004**
(21) Anmeldenummer: 00964131.7
(22) Anmeldetag: 06.09.2000
(51) Int. Cl.: A61M 35/00, A45D 34/04, A47K 3/28

(54) **EINRICHTUNG ZUM AUFTRAGEN VON FLÜSSIGEN KÖRPERPFLEGEMITTELN, INSBESONDERE SONNENSCHUTZEMULSIONEN**
DEVICE FOR APPLYING LIQUID BODY CARE PRODUCTS, ESPECIALLY SUNSCREEN EMULSIONS
DISPOSITIF POUR APPLIQUER DES AGENTS DE SOINS CORPORELS LIQUIDES, NOTAMMENT DES EMULSIONS DE PROTECTION SOLAIRE

(30) Priorität: 07.09.1999 DE 19942705
(43) Veröffentlichungstag der Anmeldung: 19.06.2002
(73) Patentinhaber: Von Halem, Wilhelm, 26524 Hage (DE)
(72) Erfinder: Von Halem, Wilhelm, 26524 Hage (DE)
(74) Vertreter: Patentanwälte Leinweber & Zimmermann
(86) Internationale Anmeldenummer: PCT/EP2000/008700
(87) Internationale Veröffentlichungsnummer: WO 2001/017603

(56) Entgegenhaltungen:
- BE-A- 814 319
- CH-A- 167 063
- DE-A- 19 721 373
- DE-C- 4 325 971
- DE-U- 9 319 158
- US-A- 5 387 200
- US-A- 5 829 072

## Beschreibung

Die Erfindung betrifft eine Einrichtung zum Auftragen von flüssigen Körperpflegemitteln, insbesondere Sonnenschutzemulsionen, auf die Haut eines menschlichen Körpers mit mindestens einem zur Aufnahme des Körperpflegemittels dienenden Vorratsbehälters und mindestens einer zum Abgeben des Körperpflegemittels in Richtung auf den Körper eines Benutzers betreibbaren Sprühdüsenanordnung, eine Sprühdüsenanordnung einer derartigen Einrichtung sowie ein Verfahren zum Auftragen von flüssigen Körperpflegemitteln unter Verwendung dieser Einrichtung.

Mit zunehmender Zerstörung der Ozonschicht steigt die Gefährdung der Haut infolge anwachsender Intensität der UV-Einstrahlung. Im Hinblick auf diese Gefahr nimmt die Anwendung flüssiger Körperpflegemittel in Form von Sonnenschutzemulsionen stetig zu. Dabei werden in der Regel aus einem leicht verformbaren Material, wie etwa Kunststoff, bestehende Behälter eingesetzt, die das Körperpflegemittel enthalten und bei gezielter Druckausübung die jeweils gewünschte Menge des Pflegemittels freigeben. Bei Anwendung einer derartigen bekannten Einrichtung hat es sich jedoch gezeigt, daß ein wirksamer optimaler Auftrag der Körperpflegemittel in der Regel besondere Aufmerksamkeit erfordert und das Ergebnis häufig zu wünschen übrig läßt; entweder erfolgt der Auftrag im Übermaß oder in nicht ausreichender Stärke. Darüber hinaus ist der Auftrag von Körperpflegemitteln unter Einsatz der bekannten Behälter auch noch deswegen problematisch, weil der Auftrag des Pflegemittels an schwer zugänglichen Stellen des Körpers, wie etwa am Rücken, große Schwierigkeiten bereitet und nicht immer fremde Hilfe zur Verfügung steht, um an diesen Stellen einen ausreichenden Schutz zu bewirken.

Dieses Problem ist bereits in dem deutschen Gebrauchsmuster G 87 14 652.5 angesprochen. Zur Lösung dieses Problems wird in der genannten Schrift der Einsatz einer Verteileinrichtung vorgeschlagen, die an einer Standsäule angebracht ist und mindestens im wesentlichen horizontal angeordnete absperrbare Öffnungen aufweist. Dabei kann die Säule in einer Kabine angeordnet sein, die die Pflegesuchenden vor neugierigen Blicken schützt. Bei der bekannten Vorrichtung wird die effektive Sprühfläche durch die Anzahl der Sprühdüsen bestimmt, wobei diese Sprühdüsen ein- oder mehrzeilig in vertikaler Richtung an der Säule angeordnet sein können. Durch Betätigen entsprechender Absperrorgane für die einzelnen Sprühdüsen kann die Sprühfläche definiert eingestellt werden. Dabei ist insbesondere daran gedacht, die Sprühhöhe in Abhängigkeit von der Körpergröße des Pflegesuchenden zu begrenzen.

Bei Einsatz der aus der genannten Schrift bekannten Vorrichtungen hat es sich jedoch gezeigt, daß es häufig zu Fehlbedienungen kommt, welche entweder eine unzureichende Behandlung des Pflegesuchenden mit dem Körperpflegemittel zum Ergebnis haben, oder zum Besprühen der Haare, Augen oder anderer nicht zu behandelnder Körperteile führen. Ferner hat sich die in der genannten Schrift vorgeschlagene Betätigung der Absperrorgane für die einzelnen Sprühdüsen aus hygienischen Gründen als problematisch erwiesen. Dieser Mangel kann auch nicht durch eine automatische Steuerung der in der DE 195 01 014 A1 im Zusammenhang mit dem Betrieb von Duschen der beschriebenen Art gelöst werden, weil mit einer derartigen Steuerung lediglich die Anwesenheit einer Person erfaßt und ansprechend auf entsprechende Erfassungssignale der Betrieb der Sprühelnrichtung ausgelöst werden kann. Bei einer derartigen automatischen Inbetriebnahme von Vorrichtungen der eingangs beschriebenen Art kann es besonders häufig zu einer unerwünschten Abgabe von Körperpflegemitteln auf nicht zu behandeinde Körperteile kommen.

Ferner ist aus dem Deutschen Gebrauchsmuster 93 19 158 eine Kabine bekannt an deren Innenwand Sprühdüsen von Flüssigkeitsbehältem für Hautbehandlungsmittel vorgesehen sind, wobei diese Sprühdüsen mit einem Steuerelement für den quantitativen Düsenaustrag verbunden sind. Dabei kann die Steuerung der Menge des abzugebenden Hautbehandlungsmittels in Abhängigkeit von dem Gewicht und/oder der Größe des Benutzers erfolgen.

Angesichts dieser Probleme im Stand der Technik liegt der Erfindung die Aufgabe zugrunde eine Einrichtung der eingangs beschriebenen Art bereitzustellen, mit der eine optimale Dosierung und Verteilung der aufzutragenden Körperpflegemittel auf die Körperoberfläche ohne hygienische Probleme bequem erreichbar ist

Diese Aufgabe wird durch die in Anspruch 1 angegebene Weiterbildung der bekannten Einrichtungen gelöst.

Diese Erfindung beruht auf der Erkenntnis, daß die bei den vorstehend erläuterten bekannten Einrichtungen auftretenden Probleme hinsichtlich der fehlerhaften Aufbringung des Körperpflegemittels in erster Linie auf Fehlbedienungen der verstellbaren Sprühdüsen zurückzuführen sind. Eine derartige Fehlbedienung kann unter Einsatz erfindungsgemäßer Einrichtungen ausgeschlossen werden, weil damit die Lage der zu behandelnden Hautfläche automatisch erfaßt und eine entsprechende Ansteuerung der Sprühdüsenanordnung durchgeführt wird, so daß es für den Ausschluß eines fehlerhaften Körperpflegemittelauftrages nicht mehr auf die Fähigkeiten des einzelnen Benutzers ankommt. Dabei kann die Lage der zu behandelnden Hautflächen in der Regel berührungslos eifaßt werden, so daß es auch nicht zu hygienischen Problemen kommt. Sobald die Lage der zu behandelnden Hautflächen erfaßt ist, wird die Sprühdüsenanordnung so angesteuert, daß die räumliche Verteilung der davon abgegebenen Körperpflegemittel einerseits eine zufriedenstellende Behandlung der Hautflächen gewährleistet und andererseits eine Verteilung dieser Hautpflegemittel auf nicht zu behandelnden Körperteilen, wie etwa dem Kopf und insbesondere der Haare, zuverlässig ausgeschlossen ist Demnach zeichnet sich ein erfindungsgemaßes Verfahren zum Auftragen von flüssigen Körperpflegemitteln, insbesondere Sonnenschutzemulsionen auf die Haut eines Benutzers im wesentlichen dadurch aus, daß in einem ersten Schritt die Lage der mit dem Körperpflegemittel zu behandelnden Hautflächen erfaßt und in einem zweiten Schritt die Abgabe des Körperpflegemittels in Richtung auf einen Benutzer in Abhängigkeit von der in dem ersten Schritt ermittelten Lage der zu behandelnden Hautfläche gesteuert wird.

Die vorstehend bereits angesprochene berührungslose Erfassung der Lage der zu behandelnden Hautflächen kann erreicht werden, wenn die Steuereinrichtung eine zum Empfangen von von der zu behandelnden Haut ausgehenden Signalen, wie etwa Ultraschallsignalen und/oder elektromagnetischen Signalen, insbesondere Licht, ausgelegte Empfängeranordnung aufweist. In diesem Fall ist die Steuereinrichtung zweckmäßigerweise mit einer zum Auswerten der von der Empfängeranordnung empfangenen Signale betreibbaren Auswertungsschaltung ausgestattet, mit der auf Grundlage des Ergebnisses der Auswertung Ansteuersignale für die Sprühdüsenanordnung erzeugt werden. Auf diese Weise kann eine vollautomatische Steuerung der erfindungsgemäßen Einrichtung verwirklicht werden.

Bei einer im besonderen für den Einsatz von zum Empfangen von Ultraschallsignalen ausgelegten Empfängeranordnungen gedachten Ausführungsform ist die Auswertungsschaltung zum Ermitteln der Entfernung zwischen dem Empfänger und dem Ort, von dem die empfangenen Signale ausgehen, betreibbar. Auf diese Weise kann mit der Auswertungsschaltung festgestellt werden, ob die empfangenen Signale von einer sich in der Nähe der Sprühdüsenanordnung aufhaltenden Person erzeugt wird oder von einer weiter entfernt liegenden Person bzw. einem weiter entfernt liegenden Gegenstand. Daher kann mit dieser Auswertungsschaltung sowohl die Anwesenheit einer zu behandelnden Person, als auch die Lage dieser Person erfaßt werden, wenn zusätzlich zu der Entfernung zwischen dem Empfänger und dem Ort, von dem die empfangenen Signale ausgehen, auch noch die Einfallsrichtung der empfangenen Signale erfaßt wird.

Im besonderen für den Einsatz der erfindungsgemäßen Einrichtung zum Behandeln von Personen, die während der Behandlung auch noch Kleidungsstücke tragen, hat es sich als besonders zweckmäßig erwiesen, wenn die Auswertungsschaltung zum Ermitteln von Oberflächeneigenschaften, insbesondere der Farbe, des Ortes, von dem die Signale ausgehen, betreibbar ist. Dann kann mit Hilfe der Auswertungsschaltung ermittelt werden, ob es sich bei dem Ort, von dem die Signale ausgehen, um eine freiliegende Hautfläche oder um ein Kleidungsstück handelt und die Ansteuerung der Sprühdüsenanordnung so erfolgen, daß die Kleidungsstücke nicht mit dem Körperpflegemittel besprüht werden. Bei dieser Ausführungsform der Erfindung kann die Empfängeranordnung beispielsweise ein Halbleiterelement, insbesondere eine Photodiode aufweisen, dessen Ausgangssignal bei geeigneter Wahl der Bandstruktur des Halbleitermaterials sowohl von der Intensität einfallender Lichtsignale, als auch von deren Farbe bzw. Wellenlänge abhängt. Dabei ist dem Halbleitermaterial zweckmäßigerweise mindestens eine Linsenanordnung vorgeschaltet, mit der das aus einer vorgegebenen Richtung einfallende Licht auf der Photodiode fokussiert wird. In diesem Fall kann eine besonders gute räumliche Auflösung und damit auch eine besonders genaue Erfassung der Lage der zu behandelnden Hautflächen erzielt werden.

Wie vorstehend bereits erläutert, weist die Empfängeranordnung der erfindungsgemäßen Einrichtung zweckmäßigerweise mindestens einen Ultraschallempfänger und/oder eine Photodiode auf. Daneben ist jedoch auch an den Einsatz von Empfängeranordnungen in Form von Kameras, insbesondere digitalen Kameras gedacht, mit denen ein vollständiges Bild der zu behandelnden Person erzeugt werden kann. Die Lage der zu behandelnden Haut kann bei Einsatz derartiger Kameras mit Hilfe eines geeigneten Bilderverarbeitungssystems festgestellt werden.

Wenngleich die erfindungsgemäße Einrichtung auch sinnvoll betrieben werden kann, wenn die von der Haut ausgehenden Signale in Form von von der Haut reflektiertem Sonnenlicht, Wärmestrahlung od.dgl. vorliegen, hat es sich im besonderen für den Erhalt einer besonders guten räumlichen Auflösung bzw. Zuordnung der erfaßten Signale zu definierbaren Flächen als besonders günstig erwiesen, wenn die Steuereinrichtung einen Sender zum Abgeben von Signalen in Richtung auf den Körper des Benutzers, wie etwa einen Ultraschallsender und/oder einen Sender zum Abgeben von elektromagnetischen Signalen, insbesondere Licht, wie etwa eine Glühbime oder einen Laser aufweist Unter Einsatz derartiger Sender kann festgelegt werden, welche Raumbereiche überhaupt ausgeleuchtet werden, so daß anhand der von der Empfängeranordnung erfaßten Signale sogleich festgestellt werden kann, woher diese Signale stammen. In diesem Zusammenhang hat es sich als besonders günstig erwiesen, wenn der Sender zum Abgeben der Signale in nur einer ggf. veränderbaren Raumrichtung ausgelegt ist. Diese Voraussetzung ist bei Ultraschallsendern oder Lasern in der Regel ohne weiteres gegeben. Bei Einsatz eines Senders in Form einer einfachen Glühbime kann die Abstrahlung von Licht in nur eine Raumrichtung dadurch erreicht werden, daß der Glühbime eine entsprechende Kollimatorlinse zugeordnet wird, mit der das von der Glühbime abgegebene Licht in ein sich nur in einer Raumrichtung ausbreitendes Lichtstrahlenbündel umgewandelt wird. In allen Fällen kann die Veränderung der Ausbreitungsrichtung der von dem Sender abgegebenen Signalen besonders einfach bewirkt werden, wenn der Sender ggf. zusammen mit dem Empfänger um mindestens eine vorzugsweise etwa horizontal veriaufende Achse, verschwenkbar gelagert ist Die gemeinsame Bewegung von Sender und Empfänger ist im besonderen deswegen zweckmäßig, weil dadurch die räumlich Beziehung zwischen diesen Elementen unverändert bleibt, so daß die Änderungen der von der Empfängeranordnung empfangenen Signale ausschließlich von den Änderungen der Reflektionseigenschaften im Bereich der sich ausbreitenden Signale hervorgerufen werden, so daß bei der Auswertung dieser empfangenen Signale keine Nebeneffekte berücksichtigt werden müssen.

In baulicher Hinsicht ist die erfindungsgemäße Erfindung besonders zweckmäßig, weil die Sprühdüsenanordnung mindestens eine bewegbare, insbesondere um mindestens eine vorgegebene Achse verschwenkbar gelagerte Sprühdüse aufweist, weil mit einer derartigen Sprühdüsenanordnung unter Verwendung von nur einer Sprühdüse viele bezüglich der Sprühdüsenanordnung unterschiedlich positionierte Hautflächen behandelt werden können, indem die Sprühdüse entsprechend bewegt bzw. verschwenkt wird. Bei dieser Ausführungsform hat es sich als besonders günstig erwiesen, wenn die Sprühdüse zusammen mit dem Sender und/oder Empfänger um eine gemeinsame Schwenkachse verschwenkbar gelagert ist, weil dann bei Empfang der von der zu behandelnden Haut ausgehenden Signale die zur Behandlung dieser Hautflächen benötigte Stellung der Sprühdüse besonders einfach ermittelt werden kann. Dabei ergibt sich eine weitere Vereinfachung, wenn der Sender zum Abgeben eines sich längs einer Einhüllenden, vorzugsweise längs des oberen Randes der Einhüllenden, des von der Sprühdüse abgegebenen Sprühkegels des Körperpflegemittels ausbreitenden Signals ausgelegt ist, weil dann die Stellung der Sprühdüse, bei der ein von dem Sender abgegebenes und von der zu behandeinden Haut reflektiertes Signal mit der Empfängeranordnung erfaßt wird, sogleich der Stellung entsprechend, bei der die Sprühdüse diese Hautfläche behandeln kann.

Wie eingangs bereits erläutert muß in der Regel sichergestellt werden, daß weder das Gesicht noch die Haare des Benutzers mit dem Körperpflegemittel besprüht werden. Eine entsprechende Steuerung der Sprühdüsenanordnung kann besonders einfach verwirklicht werden, wenn der Sender zum Abgeben von sich in einer den Körper des Benutzers in einer beispielsweise durch entsprechende Markierungen, insbesondere Fußmarkierungen, vorgegebenen Position neben dem Kopf im Schulterbereich schneidenden Vertikalebene ausbreitenden Signalen betreibbar Ist Bei dieser Anordnung des Senders kann durch eine Verschwenkung des Senders um eine horizontal verlaufende Achse der Körper des Benutzers in der entsprechenden Vertikalebene abgetastet werden, um so die Schulterhöhe des Benutzers über eine Änderung des von der zu dem Sender gehörenden Empfängeranordnung empfangenen Signals zu ermitteln. Sobald die Schulterhöhe auf diese Weise ermittelt wurde, kann die Sprühdüsenanordnung so angesteuert werden, daß das Körperpflegemittel nur noch in einen unterhalb der Schulterhöhe liegenden Raumbereich abgegeben wird.

Mit der erfindungsgemäßen Vorrichtung kann ein vollautomatischer Betrieb sichergestellt werden, weil eine von der Steuereinrichtung ansteuerbare Antriebseinrichtung zum Bewegen, insbesondere Verschwenken der Sprühdüse vorgesehen ist Wenngleich diese Antriebseinrichtung auch in Form eines pneumatischen oder hydraulischen Antriebs verwirklicht werden kann, hat es sich in baulicher Hinsicht als besonders günstig erwiesen, wenn die Antriebseinrichtung einen vorzugsweise über ein Getriebe, eine Kupplung; eine vorzugsweise parallel zur Schwenkachse verlaufende Antriebsachse und/oder einen an die Antriebsachse gekoppelten Hebelmechanismus mit mindestens einem quer zur Antriebsachse erstreckenden Hebel an die Sprühdüse gekoppelten Motor aufweist. Dabei wird der an die sich parallel zur Schwenkachse erstreckende Antriebsachse gekoppelte Hebelmechanismus vorzugsweise dann eingesetzt, wenn im Bereich der Sprühdüse selbst nur wenig Bauraum zur Verfügung steht und/oder eine Beschädigung der Antriebseinrichtung in der Nähe der Sprühdüsenanordnung befürchtet werden muß, well durch Einsatz eines Hebelmechanismus mit mindestens einem sich quer zur Antriebsachse erstreckenden Hebel die Drehbewegung der Antriebsachse in eine Schwenkbewegung der Sprühdüse um eine mit Abstand parallel von der Antriebsachse verlaufende Schwenkachse umgesetzt werden kann. Darüber hinaus ermöglicht der Einsatz eines geeigneten Hebelmechanismus zum Übertragen der Drehbewegung von der Antriebsachse auf die Sprühdüse bei geeigneter Wahl der Hebellängen und Ankopplungspunkte auch noch eine Umsetzung der Drehbewegung in eine Horizontal- und/oder Vertikalbewegung der Sprühfläche auf dem Körper des Benutzers mit einer im wesentlich konstanten Geschwindigkeit. Dadurch kann ein besonders gleichmäßiger Auftrag des Körperpflegemittels sichergestellt werden.

Im Sinne eines besonders gleichmäßigen Auftrags des Körperpflegemittels hat es sich ferner als besonders zweckmäßig erwiesen, wenn die Sprühdüsenanordnung nach Art eines Zerstäubers ausgeführt ist und mindestens eine Druckleitung zum Zuführen eines unter Druck stehenden Trägermediums. wie etwa Preßluft, und mindestens eine im Bereich der Sprühdüse in diese Druckleitung mündende Zuführeinrichtung für das flüssige Körperpflegemittel aufweist, weil in diesem Fall das Körperpflegemittel mit dem Trägermedium fein zerstäubt wird und so aus der Sprühdüse austritt.

Die Wahl der bevorzugten Körperpflegemittel, insbesondere Sonnenschutzemulsionen, hängt in der Regel von den persönlichen Neigungen der einzelnen Benutzer ab. Aus diesem Grund weist die Zuführeinrichtung der erfindungsgemäßen Einrichtung vorzugsweise mindestens zwei Zuführleitungen zum selektiven Zuführen von vorzugsweise aus mindestens zwei Vorratsbehältem entnommenen Körperpflegemitteln auf, um so eine Wahlmöglichkeit für die Benutzer zum Auswählen bevorzugter Körperpflegemittel bereitzustellen. Dazu ist die Zuführeinrichtung zweckmäßigerweise mit einer die selektive Zuführung der Körperpflegemittel zur Sprühdüse ermöglichenden Ventilanordnung ausgestattet. Konstruktiv hat es sich als besonders günstig erwiesen, wenn die Zuführleitungen in eine gemeinsame Kammer münden, aus der das Körperpflegemittel über eine weitere, in die Druckleitung mündende Zuführleitung entnommen werden kann. Dabei kann zwischen der Behandlung mit unterschiedlichen Körperpflegemitteln eine besonders einfache Entfernung der zuvor benutzten Körperpflegemittel aus der Kammer und/oder der weiteren Zuführleitung erfolgen, wenn die Zuführeinrichtung auch noch eine Sekundärdruckleitung zum selektiven Zuführen eines unter Druck stehenden Mediums, insbesondere Preßluft in die gemeinsame Kammer aufweist. Mit dem über die Sekundärdruckleitung zugeführten Medium können die Rückstände des zuvor benutzten Körperpflegemittels aus der Kammer und der weiteren Zuführleitung ausgeblasen werden.

Bei der gerade beschriebenen Sprühdüsenanordnung mit einer Druckluftleitung und einer Zuführeinrichtung für die Körperpflegemittel kann eine Bewegung der Sprühdüse, insbesondere eine Schwenkbewegung, unter Vermeidung von Bewegungen großer Bauelemente besonders einfach verwirklicht werden, wenn die Druckleitung und/oder mindestens eine der Zuführleitungen einen ersten, vorzugsweise etwa parallel zur Schwenkachse verlaufenden Abschnitt und einen fluiddicht damit verbundenen, an seinem dem ersten Abschnitt entgegengesetzten Ende die Sprühdüse aufweisenden und bezüglich dem ersten Abschnitt bewegbaren, vorzugsweise verschwenkbar gelagerten, zweiten Abschnitt aufweist, wobei die Antriebseinrichtung an den zweiten Abschnitt gekoppelt ist. Dabei hat es sich als besonders vorteilhaft erwiesen, wenn die Zuführeinrichtung eine Anzahl von in den zweiten Abschnitt eingeführten flexiblen Schläuchen aufweist, welche in eine in den zweiten Abschnitt der Druckleitung feststehend angeordnete gemeinsame Kammer münden, aus der das Körperpflegemittel dann über eine weitere ebenfalls in dem zweiten Abschnitt der Druckleitung feststehend angeordnete Zuführleitung entnommen wird, wobei diese weitere Zuführleitung in der Nähe der Sprühdüse in die Druckleitung mündet. Dabei weist der zweite Abschnitt zweckmäßigerweise eine Krümmung auf, längs der das unter Druck stehende Trägermedium um einen Winkel von vorzugsweise etwa 90° umgelenkt wird. Die Sprühdüsenanordnung der erfindungsgemäßen Einrichtung kann besonders einfach an einem Träger angebracht werden, wenn sie zwei sich etwa senkrecht zum ersten Abschnitt der Druckleitung erstreckender Halteelemente aufweist, zwischen denen der zweite Abschnitt angeordnet ist, wobei eines der Halteelemente von der Druckleitung und das andere von einem Antriebselement der Antriebseinrichtung durchsetzt ist.

Zusätzlich oder alternativ zu der gerade beschriebenen und zum Zerstäuben des Körperpflegemittels dienenden Druckleitung kann die erfindungsgemäße Sprühdüsenanordnung auch noch mindestens ein vorzugsweise durch eine Druckpumpe, besonders bevorzugt eine Kolbenpumpe, gebildetes Druckgerät aufweisen, mit dem das Körperpflegemittel über mindestens eine Verbindungsleitung aus dem Vorratsbehälter zu der Sprühdüse befördert wird. Dabei ist es nicht zwingend erforderlich, daß die Druckpumpe im Vorratsbehälter zur unmittelbaren Druckbeaufschlagung der Emulsionsoberfläche vorgeschaltet ist. Vielmehr kann die Druckpumpe auch in die Verbindungsleitung vom Vorratsbehälter zur Sprühdüse eingefügt sein. Zusätzlich oder alternativ zu der Druckpumpe kann das Körperpflegemittel auch pneumatisch mit Hilfe eines das Körperpflegemittel in dem Vorratsbehälter mit Druck beaufschlagenden Druckgasspeichers in Richtung auf die Sprühdüse gefördert werden.

Die erfindungsgemäße Einrichtung könnte im Freien, und zwar in der Regel im Strandbereich, an einer Wand, einer Tragsäule od.dgl. festgelegt sein. Als besonders zweckmäßig hat es sich jedoch erwiesen, wenn zumindest die Sprühdüsenanordnung der erfindungsgemäßen Einrichtung zumindest teilweise in einer Sprühkabine angeordnet ist. Auf diese Weise ist eine den Blicken der Allgemeinheit entzogene diskrete Anwendung der Einrichtung möglich. Außerdem läßt sich die Einrichtung dann auch während der Nachtzeit durch Abschließen der Kabinentür gegen Betreten bzw. Benutzung durch unbefugte oder gar gegen Vandalismus schützen.

Wie vorstehend bereits im einzelnen erläutert, hat es sich im Sinne eines gleichmäßigen Auftrags der Körperpflegemittel mit Hilfe einer konstruktiv einfachen Sprühdüsenanordnung als zweckmäßig erwiesen, wenn mindestens eine der Sprühdüsen dieser Sprühdüsenanordnung beweglich, insbesondere schwenkbar gelagert ist. Weiter hat es sich in diesem Zusammenhang als besonders günstig erwiesen, wenn die Sprühdüsenanordnung mindestens zwei zweckmäßigerweise übereinander angeordnete, vorzugsweise jeweils schwenkbar gelagerte Sprühdüsen aufweist. Darüber hinaus kann ein gleichmäßiger Auftrag des Körperpflegemittels auf alle zu behandelnden Hautflächen erreicht werden, wenn die Sprühkabine eine vorzugsweise mit einem motorischen Antrieb ausgerüstete, um eine vertikale Achse drehbar gelagerte Stützscheibe aufweist. Der auf der Stützscheibe stehende Benutzer sorgt bei seiner so erreichbaren Drehbewegung für eine gleichmäßige Aufbringung der Sonnenschutzemulsion. Wobei mit Hilfe des motorischen Antriebs der Stützscheibe für eine gleichförmige Drehbewegung gesorgt werden kann. Mit Hilfe der vorzugsweise speicherprogrammierbar ausgeführten Steuereinrichtung der erfindungsgemäßen Einrichtung ist es in einer besonders bevorzugten Ausführungsform nicht nur möglich, die Sprührichtung unter Berücksichtigung der zuvor ermittelten Lage der zu behandelnden Haut bezüglich der Sprühdüsenanordnung einzustellen, sondern auch die Sprühintensität dem jeweiligen Bedarf entsprechend zu steuern.

Wie aus der vorstehenden Erläuterung der erfindungsgemäßen Einrichtung hervorgeht, zeichnet sich die zur Verwirklichung dieser Einrichtung einsetzbare Sprühdüsenanordnung im wesentlichen dadurch aus, daß sie mindestens eine bewegbare, vorzugsweise verschwenkbar gelagerte Sprühdüse und/oder einen Zerstäuber mit einer Druckleitung und mindestens eine in diese Druckleitung mündenden Zuführleitung für das Körperpflegemittel aufweist, wobei der bewegbaren Sprühdüse zweckmäßigerweise eine in Abhängigkeit von der mit Hilfe der Steuereinrichtung ermittelten Lage der zu behandelnden Hautflächen ansteuerbare Antriebseinrichtung zugeordnet ist.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine schematische Außenansicht einer mit einer erfindungsgemäßen Einrichtung ausgerüsteten Sprühkabine,
- Fig. 2: eine Vertikalschnittansicht der Kabine nach Fig. 1,
- Fig. 3: eine Draufsicht auf die Kabine nach Fig. 1,
- Fig.4: eine schematische Ansicht einer zur Anwendung gelangenden Sprühdüsenanordnung, teilweise im Schnitt, gemäß einer ersten Ausführungsform der Erfindung,
- Fig. 5: eine schematische Darstellung eines Sensors einer Steuereinrichtung der erfindungsgemäßen Einrichtung,
- Fig. 6: eine zur Erläuterung der Funktionsweise des Sensors gemäß Fig. 5 dienende Darstellung,
- Fig. 7: eine Detaildarstellung eines zum Betreiben einer erfindungsgemäßen Vorrichtung einsetzbaren Sensors,
- Fig. 8: eine Schnittdarstellung einer erfindungsgemäßen Sprühdüsenanordnung,
- Fig. 9: eine Draufsicht auf die Sprühdüsenanordnung gemäß Fig. 8,
- Fig. 10: eine Seitenansicht der Sprühdüsenanordnung gemäß Fig. 8 und
- Fig. 11: eine Schnittdarstellung der Sprühdüsenanordnung gemäß Fig. 8 längs der in Fig. 10 eingezeichneten Schnittebene A-A.

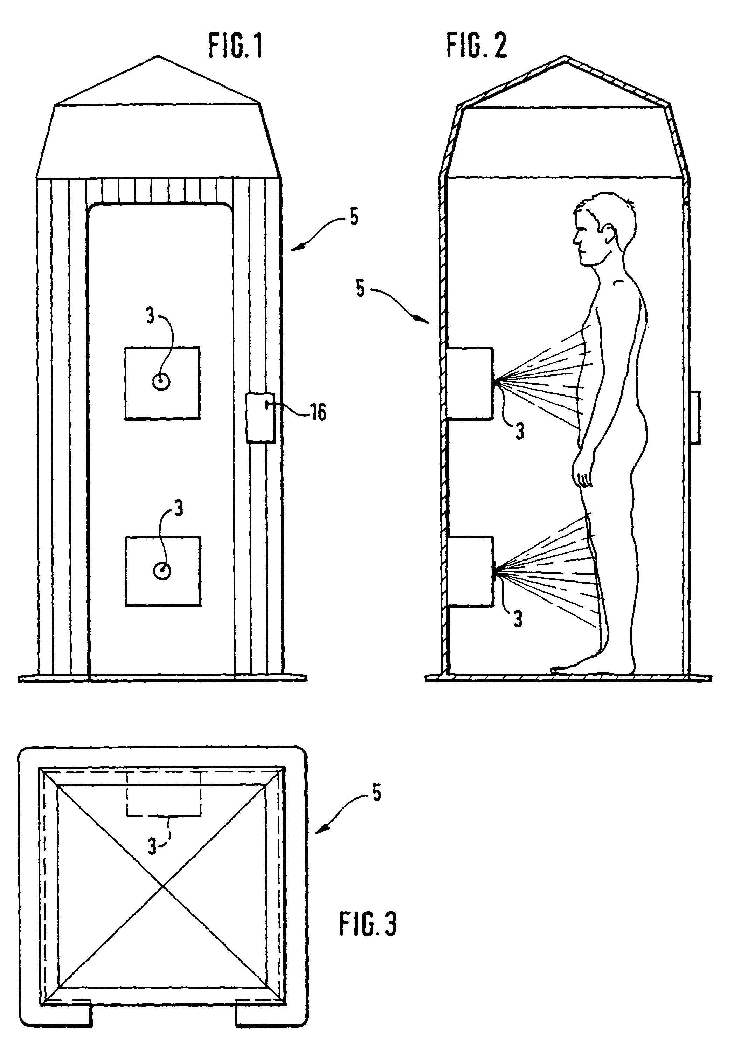

Die in den Fig. 1 bis 4 dargestellte Einrichtung zum Auftragen von Körperpflegemitteln, insbesondere Sonnenschutzemulsionen, auf die Haut eines menschlichen Körpers umfaßt mindestens ein Druckgerät 1, mindestens einen der Aufnahme eines Körperpflegemittels dienenden Vorratsbehälter 2, mindestens eine Sprühdüse 3 und eine zu letzterer führende Verbindungsleitung 4. Diese Bauelemente sind in der Praxis zu einer Sprühdüsenanordnung zusammengefaßt, die in einer Sprühkabine 5 angeordnet ist

Bei der in den Fig. 1 bis 4 dargestellten Ausführungsform ist das Druckgerät 1 durch eine Druckpumpe gebildet, die als in die Verbindungsleitung 4 zwischen Vorratsbehälter 2 und Sprühdüse 3 eingeschaltete Kolbenpumpe 6 ausgebildet ist Sie umfaßt einen von einer Kurbelwelle 7 aus über Pleuelstangen 8 angetriebenen Kolben 9, der im Kolbenzylinder 10 geführt ist. Letzterer ist einerseits über ein beim Saughub öffnendes Rückschlagventils 11 mit dem Vorratsbehälter 2 und andererseits über ein beim Druckhuböffnen des Rückschlagventils 12 mit der Sprühdüse 3 verbunden.

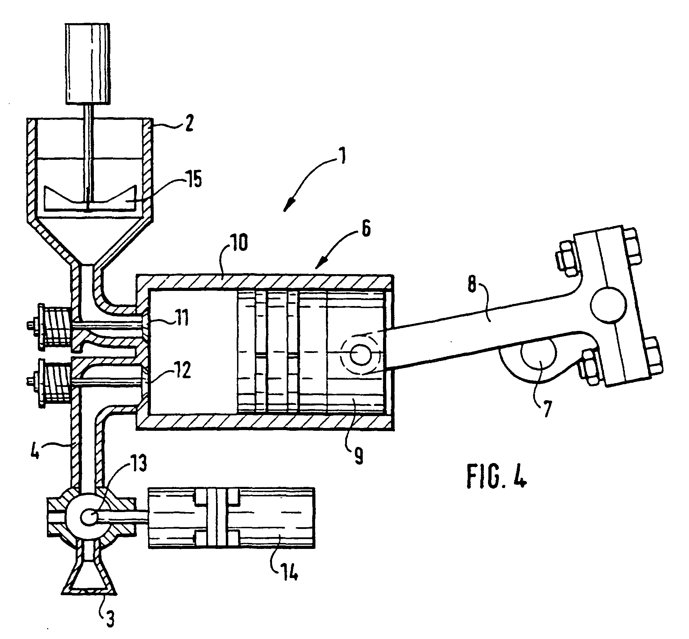

Jede Sprühdüse ist schwenkbar gelagert. In Fig. 4 ist die Schwenkachse 13 angedeutet sowie ein der Sprühdüseschwenkiagerung zugeordneter motorischer Antrieb 14.

Wie aus Fig. 2 ersichtlich sind zwei schwenkbar gelagerte Sprühdüsen 3 übereinander angeordnet.

Auf nicht näher veranschaulichte Weise kann der Sprühkabine 5 eine um eine vertikale Achse drehbar gelagerte Stützscheibe zugeordnet sein, mit deren Hilfe sich der Benutzer der Sprühkabine in Bezug auf die Sprühdüse 3 von allen Seiten gleichmäßig besprühen lassen kann. Um einen besonders gleichmäßigen Emulsionsauftrag sicherzustellen, ist die Stützscheibe mit einem motorischen Antrieb ausgestattet.

In unmittelbarer Nähe zur Sprühdüse 3 ist ein Sensor zur Erfassung der unbedeckten Hautoberfläche des Benutzers vorgesehen (siehe unten). Dieser dient der entsprechenden Steuerung der Schwenkposition der Sprühdüse 3. Dabei ist die Sprühdüsenanordnung mit einem dem Umfang der Sprühdüsenaktivität steuernden, speicherprogrammierbaren Steuerungsgerät ausgestattet. Dieses ist in der Zeichnung nicht näher dargestellt.

Der Fig. 4 ist zu entnehmen, daß jeder Vorratsbehälter 2 mit einem motorbetriebenen Rührgerät 15 ausgestattet ist. Die Kosten der erfindungsgemäßen Einrichtung können von der Gemeinschaft der Benutzer aufgebracht werden. Zu diesem Zweck ist zur Aktivierung - ggf. auch zur vorherigen Freigabe der Kabinentür - der Kabine 5 ein nicht näher veranschaulichter Automat zugeordnet, der einen Einwurfsschlitz 16 umfaßt. Anstelle von Münzen können jedoch auch Chips zur Aktivierung des Automaten Anwendung finden. Diese Möglichkeit ist mit dem Vorteil verbunden, daß derartige Chips so beschaffen sein können, daß sie die ggf. beliebig häufige Aktivierung eines Automaten über einen beim Einkauf vorbestimmten Zeitraum gestatten. Auf diese Weise ist auch die Gefahr eines Einbruchs in den Automaten beseitigt, welche bei einer Ausführung des Automaten als üblicher Geldautomat bestehen würde.

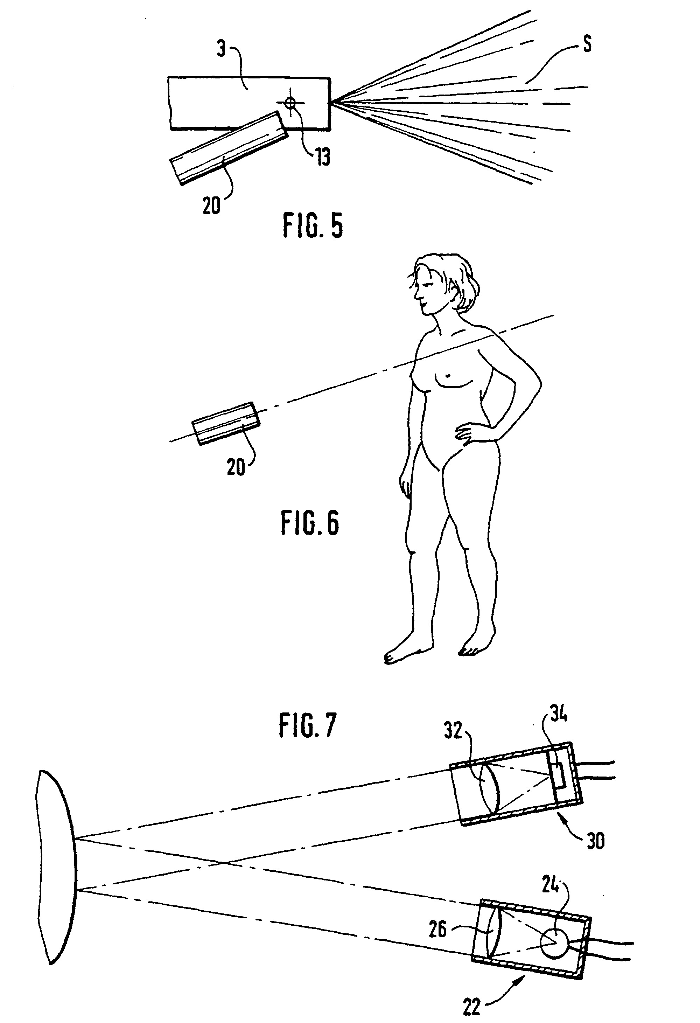

Nachstehend wird anhand der Fig. 5 und 6 das Funktionsprinzip der Steuereinrichtung der erfindungsgemäßen Einrichtung erläutert. Gemäß Fig. 5 umfaßt die Steuereinrichtung einen an einer Sprühdüse 3 festgelegten und gemeinsam mit der Sprühdüse um die horizontal verlaufende Schwenkachse 13 verschwenkbaren Sensor 20. Wie in Fig. 5 schematisch dargestellt wird mit der Sprühdüse 3 ein Sprühstrahl des Körperpflegemittels innerhalb eines Sprühstrahlkegels S abgegeben. Der Sensor 20 ist so angeordnet, daß sich die von dem Sensor 20 abgegebenen Signale längs des oberen Randes des Sprühstrahlkegels ausbreiten. Gemäß Fig. 6 trifft das von dem Sensor 20 ausgehende Signal auf die zu behandelnde Person und wird davon reflektiert. Das reflektierte Signal kann dann mit einer in dem Sensor enthaltenen Empfängeranordnung empfangen werden. Auf Grundlage der Empfangssignale kann dann die Lage der zu behandelnden Hautflächen ermittelt und die Sprühdüsenanordnung entsprechend angesteuert werden.

Dabei kann gemäß einer Ausführungsform der Erfindung der Sensor in Form eines Ultraschallsensors verwirklicht sein, mit dem Ultraschallwellen abgegeben und empfangen werden, wobei aus der Zeitdifferenz zwischen der Signalabgabe und dem Signalempfang die Entfernung zwischen dem Ultraschallsensor und dem reflektierenden Objekt ermittelt werden kann. Zur Ermittlung der Lage der zu behandelnden Hautflächen wird der Ultraschallsensor um die Schwenkachse 13 verschwenkt. Dabei wandert die obere Grenze des Sprühstrahlkegels zusammen mit dem Ultraschallstrahl auf dem Körper der zu behandelnden Person nach oben. Sobald der Ultraschallstrahl oberhalb der Schulterhöhe der zu behandelnden Person nicht mehr von dieser, sondern von einer weit dahinter liegenden Wand reflektiert wird, kann das auf Grundlage der Zeitdifferenz zwischen der Signalabgabe und dem Signalempfang erfaßt werden, so daß an diesem Punkt ein Strom- oder Spannungssprung in dem von dem Ultraschallsensor abgegebenen Signal entsteht. Dieser Strom- oder Spannungssprung kann mittels einer Schaltlogik ausgewertet und der Sprühdüsenanordnung ein der so gemessenen Schulterhöhe entsprechender maximaler Sprühwinkel zugewiesen werden. Wie besonders deutlich aus der Darstellung in Fig. 6 hervorgeht, ist der Sensor dabei so angeordnet, daß sich die davon abgegebenen Signale in einer den Körper des Benutzers in einer beispielsweise durch entsprechende Markierungen, insbesondere Fußmarkierungen, vorgegebene Position neben dem Kopf im Schulterbereich schneidenden Vertikalebene ausbreiten.

Wie aus der Darstellung in Fig. 7 hervorgeht kann im Rahmen der Erfindung auch ein zum Abgeben und Empfangen von Signalen in Form von elektromagnetischen Wellen ausgelegter Sensor eingesetzt werden. Dazu weist der Sensor einen Sender 22 mit einer gemäß Fig. 7 in Form einer Glühbime verwirklichten Lichtquelle 24 und einer Sammellinse 26 zum Erzeugen eines parallelen Lichtstrahienbündels auf. Dieses Lichtstrahlenbündel trifft im Verlauf der Schwenkbewegung des Sensors auf den Benutzer der erfindungsgemäßen Einrichtung und wird davon reflektiert. Zum Empfangen des reflektierten Lichtstrahlenbündels weist der Sensor einen insgesamt mit 30 bezeichneten Empfänger auf, welcher eine Sammellinse 32 sowie eine im Brennpunkt der Sammellinse angeordnete Photodiode 34 umfaßt. Dabei ist die Position des Empfängers 30 bezüglich dem Sender 22 festgelegt, so daß die Menge des von dem Empfänger empfangenen Lichtes nur noch von der Lage der reflektierenden Fläche bezüglich dem Sensor abhängt. Ferner wird bei der in Fig. 7 dargestellten Ausführungsform der Erfindung eine Photodiode eingesetzt, deren Bandstruktur so beschaffen ist, daß das von der Photodiode abgegebene Signal nicht nur von der empfangenen Lichtmenge sondern auch von der Wellenlänge bzw. Farbe des empfangenen Lichtes abhängt. Auf diese Weise kann mit Hilfe der Photodiode 34 das von der Haut reflektierte Licht aufgrund des Farbunterschiedes von dem von Textilien reflektierten Licht unterschieden werden. Dazu wird beim Betrieb des in Fig. 7 dargestellten Sensors zunächst ein Referenzpunkt gesucht, auf dem das charakteristische Ausgangssignal bzw. die charakteristische Ausgangsspannung oder Stromstärke ermittelt wird, die der speziellen Haut des gerade in der Sprühkabine befindlichen Menschen entspricht. Dieser charakteristische Wert wird als Referenzwert abgespeichert. Bei einer Bewegung des Sensors werden sprunghafte Abweichungen des Ausgangssignals von diesem Referenzwert, die eine bestimmte Größenordnung überschreiten, von dem zu der Steuereinrichtung gehörenden Mikroprozessor als Textiloberfläche oder als Körperende erkannt.

Auch bei dieser Ausführungsform der Erfindung ist der Sensor so an der Sprühdüse bereits befestigt, daß die Hauptachse des Lichtstrahlenbündels etwa mit der oberen Grenze des Sprühstrahlkegels der Sprühdüse zusammenfällt. Die untere Grenze des Sprühstrahlkegels ist bei Kenntnis des Sprühkegelwinkels mit Hilfe eines mit dem Mikroprozessor ausführbaren Programms ohne Schwierigkeiten ermittelbar. Bei der Ermittlung der Lage der zu behandelnden Hautfläche mit Hilfe des in Fig. 7 dargestellten Sensors wird zunächst der maximale Verschwenkwinkel des Sensors eingestellt, so daß der von dem Sensor abgegebene Lichtstrahl den höchsten Punkt der durch die Meßvorrichtung gegebenen Meßmöglichkeiten erreicht. Bei einer Verschwenkung des Sensors um die horizontal verlaufende Schwenkachse wird der Meßstrahl nach unten, also in der Richtung auf den Körper des Benutzers bewegt. Dabei tritt der erste Strom- oder Spannungssprung im Ausgangssignal des Sensors auf, sobald der Meßstrahl auf die Schulter des Benutzers trifft. Der Schwenkwinkel des Sensors 20 bei dem dieser Spannungs- oder Stromsprung auftritt kann unter weiterer Berücksichtigung des Sprühwinkels zur Ermittelung des maximalen Schwenkwinkels der Sprühdüse 3 während der Behandlung benutzt werden.

Beim Erreichen der Schulter trifft der Meßstrahl nicht zwangsläufig auf unbedeckte Haut. Vielmehr ist es auch möglich, daß der Meßstrahl zunächst auf einen Bikiniträger oder Badeanzugträger trifft. Das kann durch Schwenken des Sensors um eine vertikale Schwenkachse und eine Auswertung des von dem Sensor abgegebenen Empfangssignals überprüft werden. Sofern der Meßstrahl auf unbedeckte Haut trifft, kann dies zur Ermittlung des charakteristischen Wertes, welcher der speziellen Haut des gerade in der Sprühkabine befindlichen Benutzers entspricht, benutzt werden. Danach kann der Meßstrahl durch eine Schwenkbewegung des Sensors 20 um die Schwenkachse 13 auf der Körperoberfläche weiter nach unten bewegt werden. So kann die zu behandelnde Hautfläche unter Berücksichtigung der von dem Benutzer getragenen Bekleidungsstücke ermittelt und in einem Geometriedatenspeicher der Steuereinrichtung abgelegt werden. Diese Meßprozedur kann in verschiedenen Schnittebenen und bei verschiedenen Positionen des Benutzers bezüglich dem Sensor zur Abtastung der Vorder- und Rückseite des Benutzers wiederholt werden. Auf Grundlage der so ermittelten Geometriedaten und des bekannten Zusammenhangs zwischen der Lage des Sensors und dem Sprühkegel der Düse kann die Sprühdüsenanordnung so angesteuert werden, daß nur die zu behandelnden Hautflächen des Benutzers besprüht werden.

Bei einer weiteren Ausführungsform der Erfindung kann anstelle der Glühlampe auch ein Laser als Lichtquelle eingesetzt werden.

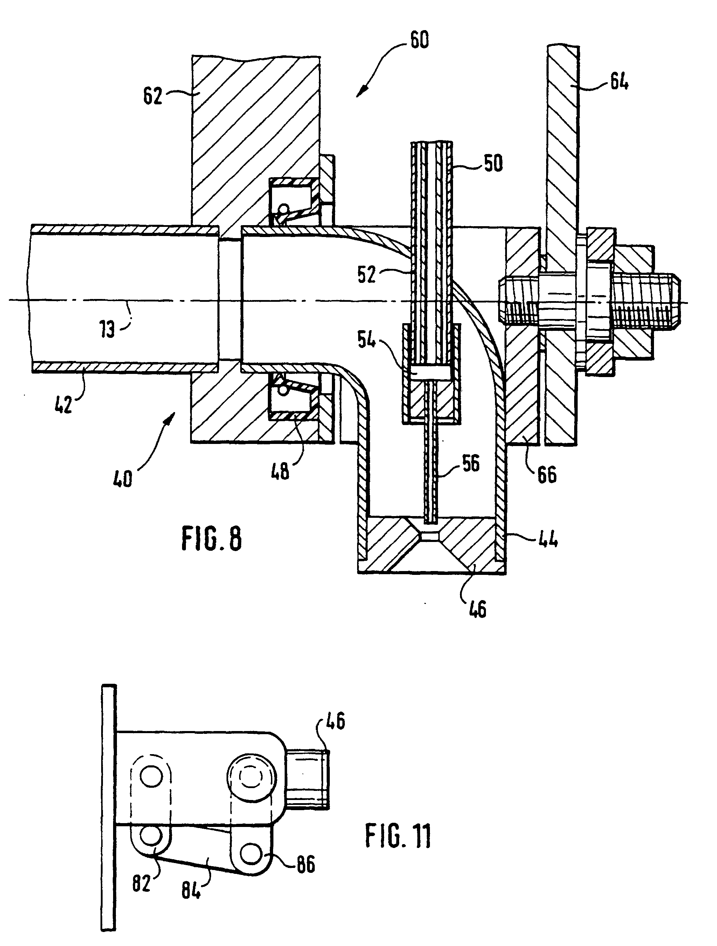

Die in den Fig. 8 bis 11 dargestellte Sprühdüsenanordnung besteht im wesentlichen aus einer insgesamt mit 40 bezeichneten Druckleitung, einer insgesamt mit 50 bezeichneten Zuführeinrichtung für das Körperpflegemittel, einer Halteeinrichtung 60, einer Antriebseinrichtung 70 und einem Hebelmechanismus 80 zum Übertragen der Motorbewegung auf die Sprühdüsenanordnung. Die Druckleitung 40 besteht aus einem ersten bezüglich eines Halteelementes 62 feststehend angeordneten Druckleitungsabschnitt 42 sowie einem über einen Dichtungsring 48 dichtend in das Halteelement 62 eingesetzten und an dem in das Halteelement 62 eingesetzten Ende mit dem ersten Druckleitungsabschnitt 42 fluchtenden zweiten Druckleitungsabschnitt 44, der in Form eines Krümmers ausgebildet wird, um die über die Druckleitung 40 zugeführte Druckluft um 90° umzulenken. An dem dem ersten Abschnitt 42 abgewandten Ende des Druckleitungsabschnitts 44 ist eine Sprühdüse 46 angeordnet.

Die Zuführeinrichtung 50 für das Körperpflegemittel umfaßt ein in den zweiten Druckleitungsabschnitt 44 eingeführtes Zuführleitungsbündel 52, welches in eine feststehend innerhalb dem zweiten Druckleitungsabschnitt 44 angeordnete Kammer 54 mündet. Aus dieser Kammer 54 kann das Körperpflegemittel über eine weitere, in der Nähe der Sprühdüse 46 in den zweiten Druckleitungsabschnitt 44 mündende Zuführleitung 56 abgeleitet werden. Mit Hilfe des über die Druckleitung 40 zugeführten Trägermediums wird das über die weitere Zuführleitung 56 aus der Kammer 54 abgeleitete Körperpflegemittel zerstäubt und innerhalb eines beispielsweise in Fig. 5 schematisch dargestellten Sprühkegels abgesprüht. Das Zuführleitungsbündel 52 umfaßt eine Mehrzahl von jeweils mit einem Vorratsbehälter für Körperpflegemittel verbundenen Zuführleitungen, wobei zwischen dem Vorratsbehältem und der Kammer 54 eine Ventilanordnung vorgesehen ist, welche die selektive Einleitung eines ausgewählten Körperpflegemittels aus dem entsprechenden Vorratsbehälter in die Kammer 54 ermöglicht. Ferner umfaßt das Zuführleitungsbündel 52 auch noch eine Sekundärluftteitung mit der Körperpflegemittelreste aus der Kammer 54 und der weiteren Zuführleitung 56 ausgeblasen werden können, um die Vorrichtung zu reinigen.

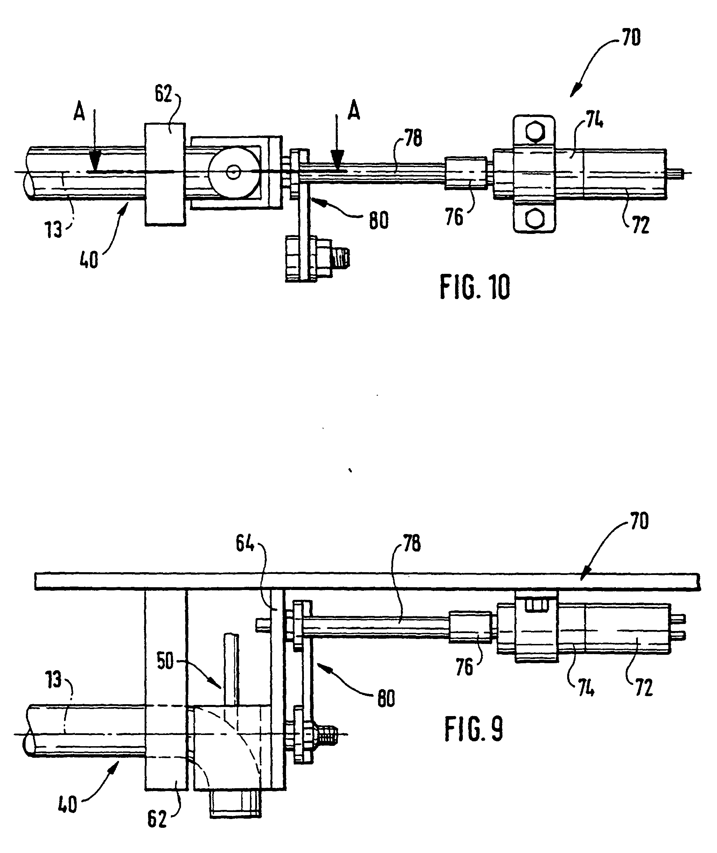

Wie besonders deutlich aus Fig. 8 hervorgeht, ist der zweite Druckleitungsabschnitt 54 zwischen dem sich etwa senkrecht zum ersten Druckleitungsabschnitt erstreckenden Halteelement 62 und einem weiteren dazu verlaufenden Halteelement 64 angeordnet, wobei dieses zweite Halteelement 64 von einem Antriebselement durchsetzt ist, mit dem der zweite Druckleitungsabschnitt 44 mit Hilfe eines Drehhalters 66 zusammen mit der Kammer 54 und der weiteren Zuführleitung 56 um die Schwenkachse 13 verschwenkt werden kann. Der Aufbau der dazu eingesetzten Antriebseinrichtung ist besonders deutlich in Fig. 10 dargestellt. Danach umfaßt die Antriebseinrichtung 70 einen Motor 72, ein Getriebe 74, eine Kupplung 76 eine Antriebsachse 78 sowie einen Hebelmechanismus 80. Der Hebelmechanismus 80 besteht im wesentlichen aus einem drehfest an die Antriebsachse 78 gekoppelten ersten Hebel 82, einer bezüglich einer parallel zur Antriebsachse 78 verlaufenden Gelenkachse gelenkig an den ersten Hebel 82 gekoppelten Schubstange 84 sowie einem einerseits an den Drehhalter 66 und andererseits bezüglich einer parallel zur Antriebsachse 78 gelenkig an die Schubstange 84 gekoppelten zweiten Hebel 86. Dieser aus dem ersten Hebel 82, der Schubstange 84 und dem zweiten Hebel 86 gebildete Hebelmechanismus ist als ungleichförmiges Getriebe ausgebildet, so daß bei einer Drehbewegung der Antriebsachse eine gleichförmige Bewegung des Sprühkegels auf dem Körper des Benutzers mit im wesentlichen konstanter Geschwindigkeit erhalten wird.

Die Erfindung ist nicht auf die anhand der Zeichnung erläuterten Ausführungsformen beschränkt. Vielmehr ist auch eine Sprühdüsenanordnung mit einer Vielzahl selektiv ansteuerbaren Sprühdüsen gedacht, von denen jede in Abhängigkeit von der mit der Steuereinrichtung ermittelten Lage der zu behandelnden Hautflächen selektiv angesteuert werden kann. Ferner ist auch an den Einsatz von Sensoren in Form von Kameras, insbesondere digitalen Kameras mit einer nachgeschalteten Bildverarbeitungseinrichtung gedacht. Des weiteren können die anhand der Fig. 4 und der Fig. 8 bis 11 dargestellten Ausführungsformen auch miteinander kombiniert werden.

## Patentansprüche

1. Einrichtung zum Auftragen von Körperpflegemitteln, insbesondere Sonnenschutzemulsionen auf die Haut eines menschlichen Körpers mit mindestens einem zur Aufnahme des Körperpflegemittels dienenden Vorratsbehälter (2) und mindestens einer zum Abgeben des Körperpflegemittels in Richtung auf den Körper eines Benutzers betreibbaren Sprühdüsenanordnung, **dadurch gekennzeichnet, daß** die Sprühdüsenanordnung mindestens eine bewegbare, insbesondere um mindestens eine vorgegebene Achse (13) verschwenkbar gelagerte Sprühdüse (3) aufweist, und die Abgabe des Körperpflegemittels mit einer Steuereinrichtung in Abhängigkeit von der Lage der mit dem Körperpflegemittel zu behandelnden Haut bezüglich der Sprühdüsenanordnung durch Ansteuern einer Antriebseinrichtung zum Bewegen, insbesondere Verschwenken der Sprühdüse steuerbar ist.

2. Einrichtung nach Anspruch 1, **dadurch gekennzeichnet, daß** die Steuereinrichtung eine zum Empfangen von von der zu behandelnden Haut ausgehenden Signalen, wie etwa Ultraschallsignalen und/oder elektromagnetischen Signalen, insbesondere. Licht- oder Wärmestrahlung, ausgelegte Empfängeranordnung aufweist.

3. Einrichtung nach Anspruch 2, **dadurch gekennzeichnet, daß** die Steuereinrichtung eine zum Auswerten der Signale betreibbare Auswertungsschaltung aufweist, mit der auf Grundlage des Ergebnisses der Auswertung Ansteuersignale für die Sprühdüsenanordnung erzeugt werden.

4. Einrichtung nach Anspruch 3, **dadurch gekennzeichnet, daß** die Auswertungsschaltung zum Ermitteln der Entfernung zwischen der Empfängeranordnung und dem Ort, von dem die empfangenen Signale ausgehen, betreibbar ist.

5. Einrichtung nach Anspruch 3 oder 4, **dadurch gekennzeichnet, daß** die Auswertungsschaltung zum Ermitteln von Oberflächeneigenschaften, insbesondere der Farbe, des Ortes, von dem die Signale ausgehen, betreibbar ist.

6. Einrichtung nach einem der Ansprüche 2 bis 5, **dadurch gekennzeichnet, daß** die Empfängeranordnung einen Ultraschallsensor und/oder eine Photodiode aufweist.

7. Einrichtung nach einem der vorhergehenden Ansprüche, **dadurch gekennzeichnet, daß** die Steuereinrichtung einen Sender zum Abgeben von Signalen in Richtung auf den Körper des Benutzers, wie etwa einen Ultraschallsender und/oder einen Sender zum Abgeben von elektromagnetischen Signalen, insbesondere Licht, aufweist.

8. Einrichtung nach Anspruch 7, **dadurch gekennzeichnet, daß** der Sender zum Abgeben der Signale in nur eine ggf. veränderbare Raumrichtung ausgelegt ist.

9. Einrichtung nach Anspruch 7 oder 8, **dadurch gekennzeichnet, daß** der Sender ggf. zusammen mit dem Empfänger um mindestens eine, vorzugsweise etwa horizontal verlaufende Achse verschwenkbar gelagert ist.

10. Einrichtung nach Anspruch 9, **dadurch gekennzeichnet, daß** die Sprühdüse zusammen mit dem Sender und/oder Empfänger verschwenkbar ist

11. Einrichtung nach Anspruch 10, **dadurch gekennzeichnet, daß** der Sender zum Abgeben eines sich längs einer Einhüllenden vorzugsweise längs des oberen Randes der Einhüllenden des von der Sprühdüse abgegebenen Sprühkegels des Körperpflegemittels ausbreitenden Signals ausgelegt ist.

12. Einrichtung nach einem der Ansprüche 7 bis 11, **dadurch gekennzeichnet, daß** der Sender zum Abgeben von sich in einer den Körper des Benutzers in einer beispielsweise durch entsprechende Markierungen, insbesondere Fußmarkierungen vorgegebenen Position des Benutzers neben dem Kopf im Schulterbereich schneidenden Vertikalebene ausbreitenden Signalen betreibbar ist.

13. Einrichtung nach einem der vorhergehenden Ansprüche, **dadurch gekennzeichnet, daß** die Antriebseinrichtung einen vorzugsweise über ein Getriebe, eine Kupplung, eine vorzugsweise parallel zur Schwenkachse verlaufende Antriebsachse und/oder einen Hebelmechanismus mit mindestens einem sich quer zur Antriebsachse erstreckenden Hebel an die Sprühdüse gekoppelten Motor aufweist.

14. Einrichtung nach einem der vorhergehenden Ansprüche, **dadurch gekennzeichnet, daß** die Sprühdüsenanordnung mindestens eine Druckleitung zum Zuführen eines unter Druck stehenden Trägermediums, insbesondere Preßluft, und mindestens eine im Bereich der Sprühdüse in diese Druckleitung mündende Zuführeinrichtung für das flüssige Körperpflegemittel aufweist.

15. Einrichtung nach Anspruch 14, **dadurch gekennzeichnet, daß** die Zuführeinrichtung mindestens zwei Zuführleitungen zum selektiven Zuführen von vorzugsweise aus mindestens zwei Vorratsbehältem entnommenen Pflegemitteln aufweist.

16. Einrichtung nach Anspruch 15, **dadurch gekennzeichnet, daß** die Zuführleitungen in eine gemeinsame Kammer münden, aus der das Körperpflegemittel über eine weitere in die Druckleitung mündende Zuführleitung entnommen werden kann.

17. Einrichtung nach einem der Ansprüche 14 bis 16, **dadurch gekennzeichnet, daß** die Zuführeinrichtung eine Sekundärdruckleitung zum Zuführen eines unter Druck stehenden Mediums, insbesondere Luft, in die gemeinsame Kammer aufweist.

18. Einrichtung nach einem der Ansprüche 14 bis 17, **dadurch gekennzeichnet, daß** die Druckleitung und/oder mindestens eine Zuführleitung, einen ersten, vorzugsweise etwa parallel zur Schwenkachse verlaufenden Abschnitt und einen fluiddicht damit verbondenen, an seinem dem ersten Abschnitt entgegengesetzten Ende die Sprühdüse aufweisenden und bezüglich dem ersten Abschnitt bewegbaren, vorzugsweise verschwenkbar gelagerten, zweiten Abschnitt aufweist, wobei die Antriebseinrichtung an den zweiten Abschnitt gekoppelt ist.

19. Einrichtung nach Anspruch 18, **dadurch gekennzeichnet, daß** der zweite Abschnitt einen Krümmer aufweist, mit dem das Trägermedium um einen Winkel von vorzugsweise etwa 90° umgelenkt wird.

20. Einrichtung nach Anspruch 18 oder 19, **dadurch gekennzeichnet, daß** die Sprühdüsenanordnung zwei sich etwa senkrecht zum ersten Abschnitt erstreckende Halteelemente aufweist, zwischen denen der zweite Abschnitt angeordnet ist, wobei eines der Halteelemente von der Druckleitung und das andere von einem Antriebselement der Antriebseinrichtung durchsetzt ist.

21. Einrichtung nach einem der vorhergehenden Ansprüche, **dadurch gekennzeichnet, daß** die Sprühdüsenanordnung mindestens ein vorzugsweise durch eine Druckpumpe, besonders bevorzugt Kolbenpumpe, gebildetes Druckgerät aufweist, mit dem das Körperpflegemittel über mindestens eine Verbindungsleitung aus dem mindestens einen Vorratsbehälter zu einer Sprühdüse befördert wird.

22. Einrichtung nach einem der vorhergehenden Ansprüche, **dadurch gekennzeichnet, daß** die Sprühdüsenanordnung zumindest teilweise in einer Sprühkabine angeordnet ist.

23. Einrichtung nach einem der vorhergehenden Ansprüche, **dadurch gekennzeichnet, daß** die Sprühdüsenanordnung mindestens zwei übereinander angeordnete, vorzugsweise jeweils verschwenkbar gelagerte Sprühdüsen aufweist.

24. Einrichtung nach Anspruch 22 oder 23, **dadurch gekennzeichnet, daß** die Sprühkabine eine, vorzugsweise mit einem motorischen Antrieb ausgerüstete, um eine vertikale Achse drehbar gelagerte Stützscheibe aufweist.

25. Einrichtung nach einem der vorhergehenden Ansprüche, **dadurch gekennzeichnet, daß** mindestens ein Vorratsbehälter mit einem motorbetriebenen Rührgerät ausgestattet ist.

## Claims

1. A device for the application of body-care products, in particular sunscreen emulsions, to the skin of a human body, with at least one storage container (2) used for receiving the body-care product, and at least one spray-nozzle arrangement capable of being operated in order to dispense the body-care product in the direction towards the body of a user, **characterized in that** the spray-nozzle arrangement comprises at least one movable spray nozzle (3), in particular mounted so as to be pivotable about at least one predetermined axis (13), and the dispensing of the body-care product is controllable by a control device, in a manner dependent upon the position of the skin to be treated with the body-care product, with respect to the spray-nozzle arrangement by actuating a driving device for moving, in particular pivoting, the spray nozzle.

2. A device according to Claim 1, **characterized in that** the control device comprises a receiver device which is designed for receiving signals issuing from the skin to be treated, such as for example ultrasound signals and/or electromagnetic signals, in particular light radiation or heat radiation.

3. A device according to Claim 2, **characterized in that** the control device comprises an evaluation circuit which is capable of being operated in order to evaluate the signals and by which control signals for the spray-nozzle arrangement are generated on the basis of the result of the evaluation.

4. A device according to Claim 3, **characterized in that** the evaluation circuit is capable of being operated in order to determine the distance between the receiver device and the location from which the received signals issue.

5. A device according to Claim 3 or 4, **characterized in that** the evaluation circuit is capable of being operated in order to determine surface properties, in particular the colour, of the location from which the signals issue.

6. A device according to one of Claims 2 to 5, **characterized in that** the receiver device comprises an ultrasound sensor and/or a photodiode.

7. A device according to one of the preceding Claims, **characterized in that** the control device comprises an emitter for emitting signals in the direction towards the user's body, such as for example an ultrasound emitter and/or an emitter for emitting electromagnetic signals, in particular light.

8. A device according to Claim 7, **characterized in that** the emitter is designed to emit the signals in only one spatial direction, which is optionally variable.

9. A device according to Claim 7 or 8, **characterized in that** the emitter is mounted, optionally together with the receiver, so as to be pivotable about at least one axis, preferably extending substantially horizontally.

10. A device according to Claim 9, **characterized in that** the spray nozzle is pivotable together with the emitter and/or the receiver.

11. A device according to Claim 10, **characterized in that** the emitter is designed to emit a signal which travels along an envelope preferably along the upper edge of the envelope of the spray cone of the body-care product discharged by the spray nozzle.

12. A device according to one of Claims 7 to 11, **characterized in that** the emitter is capable of being operated in order to emit signals travelling in a vertical plane which intersects with the user's body beside the head in the shoulder region, in a position of the user predetermined for example by suitable markings, for example markings for the feet.

13. A device according to one of the preceding Claims, **characterized in that** the driving device comprises a motor coupled to the spray nozzle by at least one lever extending transversely to a driving axle preferably extending parallel to the pivot axis, preferably by way of a gear mechanism, a coupling, the driving axle and/or a lever mechanism.

14. A device according to one of the preceding Claims, **characterized in that** the spray-nozzle arrangement comprises at least one pressure line for supplying a carrier medium under pressure, in particular compressed air, and at least one supply device, opening into the said pressure line in the region of the spray nozzle, for the liquid body-care product.

15. A device according to Claim 14, **characterized in that** the supply device comprises at least two supply lines for the selective supply of body-care products removed preferably from at least two storage containers.

16. A device according to Claim 15, **characterized in that** the supply lines open into a common chamber from which the body-care product can be removed by way of a further supply line opening into the pressure line.

17. A device according to one of Claims 14 to 16, **characterized in that** the supply device comprises a secondary pressure line for supplying a medium under pressure, in particular air, into the common chamber.

18. A device according to one of Claims 14 to 17, **characterized in that** the pressure line and/or at least one supply line comprises or comprise a first portion, which preferably extends substantially parallel to the pivot axis, and a second portion, which is connected to the said first portion in a fluid-tight manner and has the spray nozzle at its end opposite the first portion and which is movable, and is preferably mounted so as to be pivotable, with respect to the first portion, wherein the driving device is coupled to the second portion.

19. A device according to Claim 18, **characterized in that** the second portion has a curve by which the carrier medium is deflected by an angle of preferably approximately 90°.

20. A device according to Claim 18 or 19, **characterized in that** the spray-nozzle arrangement comprises two holding elements which extend substantially vertically to the first portion and between which the second portion is arranged, wherein one of the holding elements is traversed by the pressure line and the other is traversed by a driving element of the driving device.

21. A device according to one of the preceding Claims, **characterized in that** the spray-nozzle arrangement comprises at least one pressure appliance, which is preferably formed by a pressure pump, and, in a particularly preferred manner, a reciprocating-piston pump, and by which the body-care product is conveyed by way of at least one connexion line from the at least one storage container to a spray nozzle.

22. A device according to one of the preceding Claims, **characterized in that** the spray-nozzle arrangement is arranged at least in part in a spraying cubicle.

23. A device according to one of the preceding Claims, **characterized in that** the spray-nozzle arrangement comprises at least two spray nozzles which are arranged one above the other and which are preferably mounted so as to be pivotable in each case.

24. A device according to Claim 22 or 23, **characterized in that** the spraying cubicle comprises a support disc which is preferably provided with a motor drive and which is mounted so as to be rotatable about a vertical axis.

25. A device according to one of the preceding Claims, **characterized in that** at least one storage container is provided with a motor-driven stirring device.

## Revendications

1. Dispositif destiné à l'application d'agents de soins corporels, notamment d'émulsions de protection contre le soleil, sur la peau d'un corps humain, comportant au moins un réservoir (2) servant à la réception de l'agent de soins corporels, et au moins un système à buse de pulvérisation pouvant fonctionner pour distribuer l'agent de soins corporels en direction du corps d'un utilisateur, **caractérisé en ce que** le système à buse de pulvérisation comporte au moins une buse de pulvérisation (3) mobile, qui est notamment montée de façon à pouvoir pivoter autour d'au moins un axe (13) prédéterminé, et **en ce que** la distribution de l'agent de soins corporels peut être commandée avec un dispositif de commande moyennant la commande d'un dispositif d'entraînement destiné à déplacer, notamment à pivoter la buse de pulvérisation par rapport au système à buse de pulvérisation, en fonction de la position de la peau à traiter avec l'agent de soins corporels.

2. Dispositif selon la revendication 1, **caractérisé en ce que** le dispositif de commande comporte un système récepteur conçu pour la réception de signaux émanant de la peau à traiter, tels que par exemple des signaux ultrasonores et/ou des signaux électromagnétiques, notamment un rayonnement lumineux ou thermique.

3. Dispositif selon la revendication 2, **caractérisé en ce que** le dispositif de commande comporte un circuit d'évaluation pouvant fonctionner pour l'évaluation des signaux, avec lequel des signaux de commande sont générés pour le système à buse de pulvérisation sur la base du résultat de l'évaluation.

4. Dispositif selon la revendication 3, **caractérisé en ce que** le circuit d'évaluation peut fonctionner pour la détermination de la distance entre le système récepteur et l'emplacement d'où proviennent les signaux reçus.

5. Dispositif selon la revendication 3 ou 4, **caractérisé en ce que** le circuit d'évaluation peut fonctionner pour la détermination de propriétés de surface, notamment de la couleur, de l'emplacement d'où proviennent les signaux.

6. Dispositif selon l'une quelconque des revendications 2 à 5, **caractérisé en ce que** le système récepteur comporte un capteur d'ultrasons et/ou une photodiode.

7. Dispositif selon l'une quelconque des revendications précédentes, **caractérisé en ce que** le dispositif de commande comporte un émetteur pour délivrer des signaux en direction du corps de l'utilisateur, tel que par exemple un émetteur d'ultrasons, et/ou un émetteur pour délivrer des signaux électromagnétiques, notamment de la lumière.

8. Dispositif selon la revendication 7, **caractérisé en ce que** l'émetteur est conçu pour ne délivrer les signaux que dans une seule direction spatiale, qui est le cas échéant variable.

9. Dispositif selon la revendication 7 ou 8, **caractérisé en ce que** l'émetteur, le cas échéant avec le récepteur, est monté de façon à pouvoir pivoter autour d'au moins un axe, qui s'étend de préférence approximativement à l'horizontale.

10. Dispositif selon la revendication 9, **caractérisé en ce que** la buse de pulvérisation peut être pivotée en même temps que l'émetteur et/ou le récepteur.

11. Dispositif selon la revendication 10, **caractérisé en ce que** l'émetteur est conçu pour délivrer un signal se propageant le long d'une enveloppe, de préférence le long du bord supérieur de l'enveloppe du cône de pulvérisation, de l'agent de soins corporels délivré par la buse de pulvérisation.

12. Dispositif selon l'une quelconque des revendications 7 à 11, **caractérisé en ce que** l'émetteur peut fonctionner en délivrant des signaux se propageant dans un plan vertical sécant dans la zone des épaules à côté de la tête de l'utilisateur, dans une position de l'utilisateur, qui est par exemple prédéterminée par des repères correspondants, notamment des repères de pieds.

13. Dispositif selon l'une quelconque des revendications précédentes, **caractérisé en ce que** le dispositif d'entraînement comporte un moteur qui est de préférence couplé à la buse de pulvérisation par l'intermédiaire d'une transmission, d'un accouplement, d'un axe d'entraînement, s'étendant de préférence parallèlement à l'axe de pivotement, et/ou d'un mécanisme à levier comportant au moins un levier s'étendant transversalement à l'axe d'entraînement.

14. Dispositif selon l'une quelconque des revendications précédentes, **caractérisé en ce que** le système à buse de pulvérisation comporte au moins une conduite sous pression pour l'amenée d'un fluide de support sous pression, notamment de l'air comprimé, et au moins un dispositif d'amenée pour l'agent de soins corporels liquide, qui débouche dans cette conduite sous pression dans la zone de la buse de pulvérisation.

15. Dispositif selon la revendication 14, **caractérisé en ce que** le dispositif d'amenée comporte au moins deux conduites d'amenée pour l'amenée sélective d'agents de soins prélevés de préférence d'au moins deux réservoirs.

16. Dispositif selon la revendication 15, **caractérisé en ce que** les conduites d'amenée débouchent dans une chambre commune, de laquelle l'agent de soins corporels peut être prélevé au moyen d'une autre conduite d'amenée débouchant dans la conduite sous pression.

17. Dispositif selon l'une quelconque des revendications 14 à 16, **caractérisé en ce que** le dispositif d'amenée comporte une conduite secondaire sous pression pour l'amenée d'un fluide sous pression, notamment d'air, dans la chambre commune.

18. Dispositif selon l'une quelconque des revendications 14 à 17, **caractérisé en ce que** la conduite sous pression et/ou au moins une conduite d'amenée comportent un premier tronçon, qui s'étend à peu près parallèlement à l'axe de pivotement et un deuxième tronçon qui y est relié de façon étanche aux fluides, dont l'extrémité opposée au premier tronçon est munie de la buse de pulvérisation, et qui est mobile par rapport au premier tronçon, de préférence disposé de façon à pouvoir pivoter, le dispositif d'entraînement étant couplé au deuxième tronçon.

19. Dispositif selon la revendication 18, **caractérisé en ce que** le deuxième tronçon comporte un coude, avec lequel le fluide de support est dévié d'un angle qui est de préférence de l'ordre de 90°.

20. Dispositif selon la revendication 18 ou 19, **caractérisé en ce que** le système à buse de pulvérisation comporte deux éléments de retenue qui s'étendent à peu près perpendiculairement au premier tronçon, entre lesquels est disposé le deuxième tronçon, l'un des éléments de retenue étant traversé par la conduite sous pression, et l'autre élément de retenue étant traversé par un élément d'entraînement du dispositif d'entraînement.

21. Dispositif selon l'une quelconque des revendications précédentes, **caractérisé en ce que** le système à buse de pulvérisation comporte au moins un appareil sous pression qui est de préférence constitué d'une pompe de refoulement, de façon particulièrement préférée d'une pompe à piston, avec lequel l'agent de soins corporels est amené à partir de l'au moins un réservoir vers une buse de pulvérisation par l'intermédiaire d'au moins une conduite de liaison.

22. Dispositif selon l'une quelconque des revendications précédentes, **caractérisé en ce que** le système à buse de pulvérisation est disposé, au moins partiellement, dans une cabine de pulvérisation.

23. Dispositif selon l'une quelconque des revendications précédentes, **caractérisé en ce que** le système à buse de pulvérisation comporte au moins deux buses de pulvérisation disposées l'une au-dessus de l'autre, qui sont de préférence respectivement montées de façon à pouvoir pivoter.

24. Dispositif selon la revendication 22 ou 23, **caractérisé en ce que** la cabine de pulvérisation comporte un plateau de support monté en rotation autour d'un axe vertical, qui est de préférence équipé d'un entraînement motorisé.

25. Dispositif selon l'une quelconque des revendications précédentes, **caractérisé en ce qu'**au moins un réservoir est équipé d'un agitateur motorisé.
